(19) Europäisches Patentamt
European Patent Office
Office européen des brevets

(11) Numéro de publication: **0 148 167**
**B1**

(12) # FASCICULE DE BREVET EUROPÉEN

(45) Date de publication du fascicule du brevet: **31.05.89**

(21) Numéro de dépôt: **85400004.9**

(22) Date de dépôt: **04.01.85**

(51) Int. Cl.⁴: **C 07 D 215/26**, C 07 D 401/12, C 07 D 417/12, A 61 K 31/495, C 07 F 9/60

(54) **Nouveaux dérivés de la quinoléine, leur procédé de préparation et les compositions pharmaceutiques les renfermant.**

(30) Priorité: **04.01.84 FR 8400058**

(43) Date de publication de la demande: **10.07.85 Bulletin 85/28**

(45) Mention de la délivrance du brevet: **31.05.89 Bulletin 89/22**

(84) Etats contractants désignés: **AT BE CH DE FR GB IT LI LU NL SE**

(56) Documents cité:
**EP-A-0 047 923**
**EP-A-0 067 772**
**EP-A-0 084 993**
**FR-A-2 190 443**
**US-A-3 362 956**
**US-A-3 444 173**

**CHEMICAL ABSTRACTS, vol. 70, no. 23, 9 juin 1969, page 325, no. 106350z, Columbus, Ohio, US; G.K. KIRIENKO et al.: "Derivatives of 8-hydroxyquinoline as possible anthelmintics, nematocides, and fungicides"**

(73) Titulaire: **ADIR, 22, rue Garnier, F-92200 Neuilly- sur- Seine (FR)**

(72) Inventeur: **Régnier, Gilbert, avenue du Plessis, F-92290 Châtenay- Malabry (FR)**
Inventeur: **Guillonneau, Claude, 21 Rue des Bergers, F-92140 Clamart (FR)**
Inventeur: **Lepagnol, Jean, 5 rue Maurice de Vlaminck, F-78400 Chatou (FR)**

(74) Mandataire: **Reverbori, Marcelle, ADIR 22 Rue Garnier, F-92200 Neuilly- sur- Seine (FR)**

**EP 0 148 167 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention à pour objet de nouveaux dérivés de la quinoléine, leur procédé de préparation et les compositions pharmaceutiques les renfermant.

Elle concerne particulièrement les dérivés de la quinoléine de formule générale

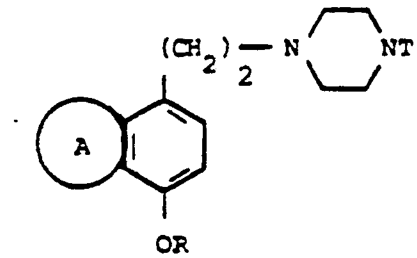

I

dans laquelle:

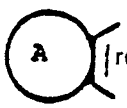 représente un groupe de formule:

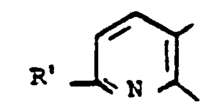

ou

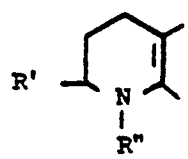

dans lesquelles:

R′ représente un atome d'hydrogène ou un radical méthyle;

R et R″, identiques ou différents représentent chacun:

un atome d'hydrogène,

un radical alcoyle ayant de 1 à 5 atomes de carbone,

un radical acyle ayant de 1 à 5 atomes de carbone,

comportant éventuellement des atomes d'halogène ou des groupes amino,

un radical alkylsulfonyle renfermant de 1 à 3 atomes de carbone, ou

un radical diéthylphosphonyle;

T est un radical méthoxy-2 phényle ou pyridyle-2.

L'état antérieur de la technique est illustré notamment par le brevet US n° 3 444 173 qui a pour objet des dérivés du 1-[5-(8 hydroxyquinolyl)]-2- aminoéthanol et les dérivés 1,2,3,4 tétrahydro correspondants, utilisables comme bronchodilatateurs, et le brevet Fr n° 2 910 443 qui concerne des dérivés du benzodioxole ayant des propriétés vasodilatatrice périphérique et stimulante du système nerveux central.

La présente invention à pour objet le procédé de préparation des dérivés de formule générale I′:

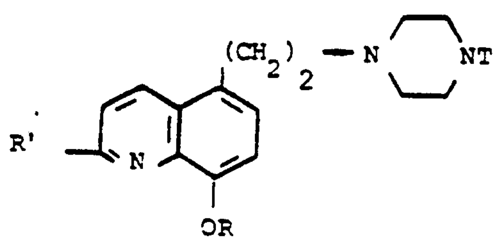

I′

dans laquelle R, R′, n et T sont tels que précédemment définis,

- caractérisé en ce que l'on condense un dérivé halogéné de formule générale II:

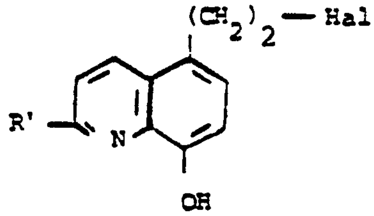

(II)

dans laquelle R' et n ont les significations précédemment définies et Hal représente un atome de chlore ou de brome, sur une pipérazine N-mono substituée de formule générale III

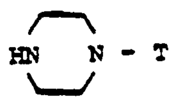

(III)

dans laquelle T a la signification définie précédemment,
- puis l'on traite le dérivé ainsi obtenu de formule générale IV:

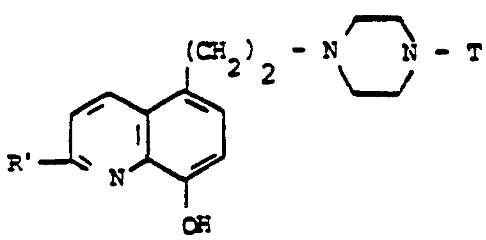

(IV)

dans laquelle R', n et T ont les significations définies précédemment,
avec un dérivé de formule générale V:

RZ (V)

dans laquelle R prend la signification définie précédemment, et Z représente, selon les valeurs de R, un atome d'halogène ou un des radicaux $-PO_3H$, COCl, $SO_2Cl$, ou $Ar-SO_3$ dans lequel Ar représente un groupe phényle éventuellement substitué par un ou plusieurs radicaux alcoyle ayant de 1 à 5 atomes de carbone.

La condensation des dérivés (II) et (III) peut être effectuée dans un solvant polaire tel que par exemple un alcool ayant de 1 à 4 atomes de carbone, ou le diméthylformamide, à une température comprise entre 60 et 120°C, en présence d'un accepteur de l'hydracide formé au cours de la réaction. Comme accepteur, on peut utiliser par exemple la triéthylamine, la pyridine, les carbonates alcalins ou un excès de la pipérazine de formule III.

Pour la transformation des composés (IV) en composés (I'), on peut employer différents réactifs RZ, selon la nature de R. Par exemple, les diéthylphosphates des dérivés (IV) peuvent être préparés par phosphatation des dérivés (IV) au moyen de $(C_2H_5O)_2-\overset{O}{\overset{\|}{P}}H$ dans un solvant aprotique ($C\,Cl_4$), à reflux, en présence de triéthylamine, selon une technique décrite dans J. Org. Chem. 42, (2), 345, (1977).

Les esters des dérivés (IV) peuvent être préparés selon une méthode classique, à partir des chlorures d'acides correspondants dans la pyridine ou le tétrahydrofuranne en présence d'une base tertiaire (triéthylamine ou pyridine), à une température comprise entre 20 et 50°C.

Les éthers des dérivés (IV) peuvent être préparés selon des méthodes classiques telles que alkylation des dérivés (IV) (à l'état de sel alcalin), au moyen d'iodure d'alkyle ou de sulfonate d'alkyle, dans un solvant polaire, comme par exemple un alcool à bas point d'ébullition ou la diméthylformamide, à une température comprise entre 60 et 90°C.

La présente invention à aussi pour objet le procédé de préparation des dérivés de formule générale I":

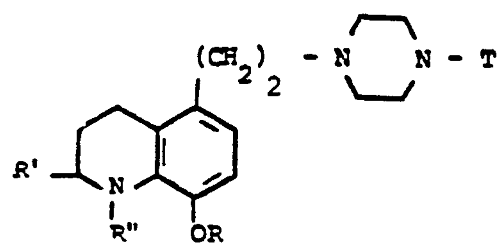

I"

dans laquelle R, R', R", n et T ont les significations précédemment definies,
- caractérisé en ce que:
l'on réduit le dérivé précédemment préparé de formule générale I':

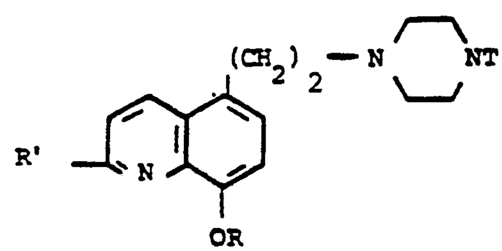

I'

dans laquelle R, R', n et T sont tels que précédemment définis, en un dérivé de formule générale II':

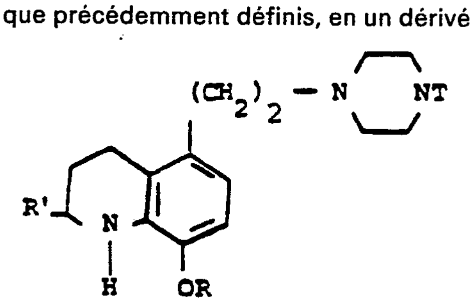

II'

que l'on traite ensuite par un réactif de formule générale III,

R" - Z (III)

dans laquelle R" et Z ont les significations précédemment définies.

Le passage du dérivé I' en dérivé II' peut être effectué par réduction à l'hydrogène en présence d'un catalyseur du 8ème groupe tel que nickel ou paladium sur charbon, dans un solvant polaire tel qu'un alcool à bas point d'ébullition, sous une pression de $3 \cdot 10^6$ à $11 \cdot 10^6$Pa, à une température comprise entre 50 et 80°C.

Les dérivés de formule I" dans laquelle la signification de R" est limitée à un radical acyle éventuellement substitué peuvent être préparés par une méthode classique analogue à celle décrite précédemment dans le procédé de préparation des dérivés I', à partir d'un halogénure d'acyle dans la pyridine ou dans le tétrahydrofuranne, en présence de triéthylamine à une température comprise entre 20 et 60°C.

Les dérivés de formule I" dans laquelle la signification de R" est limitée à un radical alcoyle, peuvent être préparés, selon des méthodes connues, par alcoylation des dérivés II' au moyen d'un iodure ou d'un sulfonate d'alcoyle dans un solvant polaire (alcool, diméthylformamide) à une température comprise entre 10 et 25°C, éventuellement en présence d'un accepteur de l'hydracide formé (triéthylamine par exemple), ou par des méthodes mettant en jeu une réduction alcoylante (HCHO - HCOOH, ou RCHO - $H_2$/Pt ou Pd/C, ou $NaBH_3OOCR$ - RCOOH) ou par réduction des amides correspondantes au moyen de $AlLiH_4$.

L'ensemble des dérivés de formule générale I' et I" forme l'ensemble des dérivés de formule générale I.

Les matières premières de formule générale II ont été préparées selon le schéma opératoire suivant, à partir des nitriles de formule (a), eux-mêmes préparés selon la méthode de V. Warner et coll., J. Med. Chem. 19, 167 - 169 (1976).

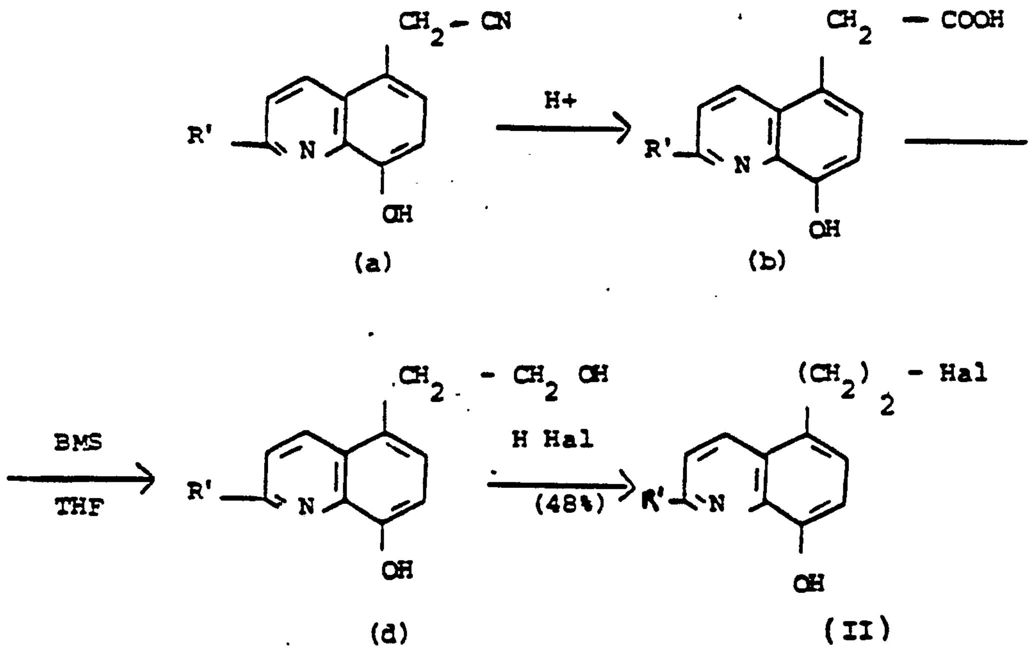


(a) (b) (d) (II)

Tous les dérivés peuvent se regrouper sous la formule générale

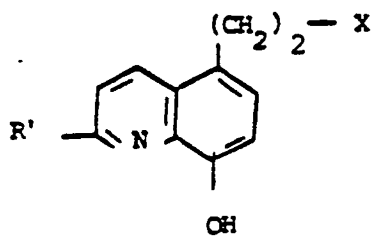

qui à été exemplifiée dans le tableau ci-après:

| R' | X | Point de fusion (Kofler) |
|---|---|---|
| H | COOH | 203°C |
| H | $CH_2OH$ | Produit résineux |
| H | $CH_2Br$ | Bromhydrate : 194 - 200°C |

Les pipérazines N-monosubstituées (III) de départ sont des produits du commerce (cf. catalogue Aldrich 1985 - 86, p. 712 et 943).

Les nouveaux dérivés de formule générale I peuvent être transformés en sels d'addition avec les acides, sels qui font, à ce titre, partie de l'invention. Comme acides utilisables pour la formation de ces sels, on peut citer, par exemple, dans la série minérale: les acides chlorhydrique, bromhydrique, sulfurique, phosphorique, et dans la série organique: les acides acétique, propionique, maléïque, fumarique, tartrique, citrique, oxalique, benzoïque, méthane sulfonique et iséthionique.

Les dérivés de formule générale I possèdent des propriétés pharmacologiques et thérapeutiques intéressantes. Ils s'opposent d'une part, aux manifestations pathologiques liées à l'accident ischémique et d'autre part aux symptômes qui accompagnent le vieillissement, notamment cérébral. Ces propriétés s'exercent de manière plus intense que celles des composés antiischémiques pris comme référence dont l'activité est reconnue depuis longtemps.

Les composés de formule générale I sont testés par leur capacité à prolonger la survie de souris soumises à un syndrome ischémique global aigu par injection intraveineuse de chlorure de magnésium. Ainsi lorsqu'ils sont administrés par voie intrapéritonéale, ces composés sont capables de prolonger significativement la survie des souris ischémiées dès la dose de 1 mg/kg comme c'est le cas par exemple pour les dérivés des exemples 14, 19, 21 et 33 décrits ci-après. Administrés per os, ils exercent de façon comparable le même effet protecteur, ce qui n'est pas le cas des substances de référence, notamment de la dihydroergotoxine qui s'avère inactive jusqu'à 10 mg/kg per os, ni de la vincamine et de l'extrait de Ginkgo biloba qui n'exercent

aucun effet aux doses inférieures à 100 mg/kg per os.

De la même façon, les composés de formule générale I présentent un effet protecteur chez la souris lors d'un syndrome hypoxique global aigu par inhalation d'azote pur. Cette action bénéfique s'exerce aux mêmes doses que lors du test précédent. Un même rapport de dose efficace est trouvé avec les substances de référence.

La dose efficace moyenne ($DE_{50}$) augmentant le temps de survie de 50 % par rapport aux animaux témoins est d'environ de 3 mg/kg lorsque ces composés sont administrés par voie intrapéritoniale ou par voie per os notamment avec les dérivés des exemples 19, 21, 30 et 33 décrits ci-après.

Sur ce test, l'index thérapeutique évalué par le rapport $DL_{50}/DE_{50}$ est très en faveur des composés de formule générale I puisqu'il est 30 fois supérieur à celui des composés de référence -vincamine, nicergoline, eburnamonine, pour la voie intrapéritonéale et plus de 5 fois supérieur pour la voie orale.

L'activité protectrice vis à vis des pertes de l'électrogénèse corticale liées au syndrome ischémique ou hypoxique est évaluée par le test d'anoxies itératives chez le rat. Cette épreuve est modifiée par rapport à sa description initiale pour la rendre plus sévère donc plus sélective. Après la seconde anoxie, alors que les animaux témoins ne récupéreront plus leur activité électrocorticale, les animaux traités par les composés, notamment les dérivés décrits dans les exemples 14, 18, 19, 21 et 33, la restaurent lorsqu'ils ont reçu par perfusion I.V. 33 micro g/kg de ceux-ci. Les substances de référence ne la restaurent que plus tardivement, à la 120ème seconde pour la Eburnamonine ou la nicergoline. Seule la dihydroergotoxine présente, puisque administrée par voie intraveineuse, une activité comparable.

Les composés de formule générale I ont été testés pour leurs éventuelles propriétés métaboliques cérébrales vis à vis d'un syndrome ischémique par hypoperfusion de 1 heure donc d'une sévérité extrême, ou vis à vis d'une imprégnation oedémateuse engendrée par le triéthyl étain per os.

Dans les deux cas, les composés exercent des activités de protection et de relance des fonctions métaboliques, puisqu'ils s'opposent totalement, ou en grande partie, à l'apparition de l'oedème. Par exemple, les dérivés des exemples 13, 18, 30 et 33 ci-après décrits, administrés chroniquement durant 4 jours à raison de 10 mg/kg, 2 fois par jour par voie intrapéritonéale, ramènent le pourcentage d'eau contenue dans le cerveau près de ou à la normale. Cette activité protectrice n'a la même intensité pour les substances de référence qu'à des doses supérieures, 5 fois plus pour la vincamine, 3 fois plus pour la nicergoline ou l'extrait de Cinkgo biloba, réputés comme puissants agents anti-oedémateux cérébraux.

Ces mêmes composés exercent leur action pharmacologique vis à vis des pertes de capacité de phosphorylation oxydative des mitochondries du cortex cérébral obtenues après un syndrome ischémique chez le rat. Ils restaurent la consommation d'oxygène de la mitochondrie stimulee par l'ADP lorsque le rat ischémié à reçu en perfusion intraveineuse 0,1 mg/kg de composé I, notamment des dérivés des exemples 14, 13 et 33 décrits ci-après. La même activité n'est obtenue pour les produits de référence qu'avec la déhydroergotoxine.

Les composés de formule générale I sont donc utilisables en traitement oral ou parentéral chez l'homme dans les cas de syndrome ischémique de toute localisation aigü, transitoire ou progressif puisqu'ils exercent leurs propriétés pharmacologiques vis à vis des nombreuses manifestations pathologiques qui accompagnent des accidents. Plus généralement, ils sont utiles dans la correction des désordres liés à l'hypoxémie et à l'insuffisance métabolique énergétique, par exemple lors du vieillissement cérébral.

La présente invention à également pour objet les compositions pharmaceutiques contenant comme principe actif un dérivé de formule générale I mélangé ou associé à un excipient pharmaceutiquement approprié. Ces compositions sont présentées sous diverses formes telles que comprimés, dragées, gélules ou préparations pour administration sublinguale, injectable ou buvable.

Le dosage unitaire peut être de 1 à 30 mg pour un traitement de 1 à 3 prises par jour.

Les exemples suivants illustrent l'invention. Les points de fusion, sauf mention contraire, ont été déterminés à la platine chauffante de Kofler.

**Exemple 1:**

{[(méthoxy-2 phényl)-4 piperazinyl] -2 éthyl}-5 hydroxy-8 quinoléine

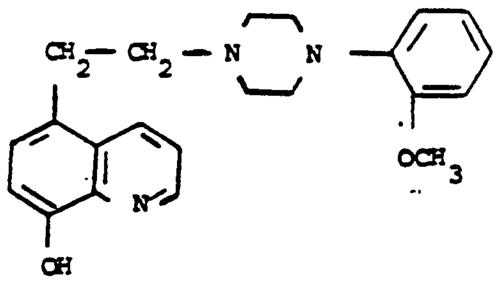

On chauffe pendant 24 heures à reflux un mélange de 50,4 g de bromoéthyl-5 hydroxy-8 quinoléine, fondant à 116°C et de 80,6 g d'O.méthoxyphényl pipérazine dans 1 l d'éthanol. Au bout de ce temps, on essore les cristaux formés, lave avec un peu d'éthanol et reprend le produit cristallisé avec 600 ml de $Na_2CO_3$ à 10 % en agitant pendant 3 heures. Ensuite, le produit est essoré, lavé à l'eau et séché sous vide, on obtient 63,5 g de {[(méthoxy-2 phényl)-4 pipérazinyl]-2 éthyl}-5 hydroxy-8 quinoléine sous forme de cristaux beiges fondant (capillaire) à 170°C.

**Exemples 2 à 4:**

Les dérivés suivants ont été préparés selon la méthode décrite dans l'exemple 1.

2) méthyl-2{[(méthoxy-2 p-hényl)-4 pipérazinyl]-2 éthyl}-5 hydroxy-8 quinoléine, P.F.: 113°C (cyclohexane).

3) {[(pyridyl-2)-4 piperazinyl]-2 éthyl}-5 hydroxy-8 quinoléine, P.F.: 147°C (eau).

4) méthyl-2{[(pyridyl-2)-4 pipérazinyl]-2 éthyl}-5 hydroxy-8 quinoléine, P.F.: 136°C (éthanol).

**Exemple 5:**

{[(méthoxy-2 phényl)-4 pipérazinyl]-2 éthyl}-5 méthoxy-8 quinoléine.

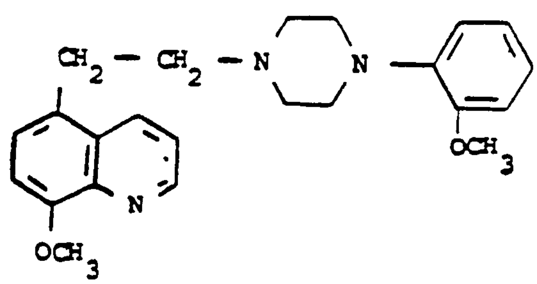

On chauffe, pendant 1 heure à 80°C, une solution de 56,4 g de {[(méthoxy-2 phenyl)-4 pipérazinyl]-2 éthyl}-5 hydroxy-8 quinoléine, dans 540 ml de diméthylformamide anhydre avec 3,72 g d'hydrure de sodium à 50 % dans l'huile. Ensuite, on ajoute 30,3 g de p.toluène sulfonate de méthyle et on chauffe le mélange 3 heures à 90°C. Au bout de ce temps, on évapore le diméthylformamide sous pression réduite et reprend le résidu avec 500 ml d'eau et 500 ml de chloroforme. On lave plusieurs fois à l'eau la solution chloroformique et évapore le chloroforme sous pression réduite. Le résidu huileux pesant 58 g est purifié par chromatoflash sur 2,2 g de silice (230 - 240 mesh) en éluant avec le mélange $CH_2Cl_2/CH_3OH$; 95/5). On obtient 46 g de {[(méthoxy-2 phényl)-4 pipérazinyl]-2 éthyl}-5 méthoxy-8 quinoléine, sous forme de produit huileux, dont le fumarate fond (capillaire) à 168°C (éthanol).

**Exemples 6 à 8:**

Les produits suivants ont été préparés selon la méthode décrite dans l'exemple 5:

6) {[(pyridyl-2)-4 pipérazinyl]-2 éthyl}-5 méthoxy-8 quinoléine, P.F.: 94°C (chlorure de méthylène).

7) méthyl-1{[(pyridyl-2)-4 pipérazinyl]-2 éthyl}-5 méthoxy-8 tétrahydro-1,2,3,4 quinoléine, P.F.: 116°C (acétate d'éthyle).

8) méthyl-1{[(méthoxy-2 phényl)-4 pipérazinyl]-2 éthyl}-5 méthoxy-8 tétrahydro-1,2,3,4 quinoléine, P.F.: 94 C (acétate d'éthyle).

D'autre part, en opérant comme dans l'exemple 5 mais en remplaçant le p.toluène sulfonate de méthyle par le p.toluène sulfonate d'éthyle, on obtient de la même façon les dérivés éthoxy-8.

**Exemple 9:**

{[(méthoxy-2 phényl)-4 pipérazinyl]-2 éthyl}-5 pivaloyloxy-8 quinoléine.

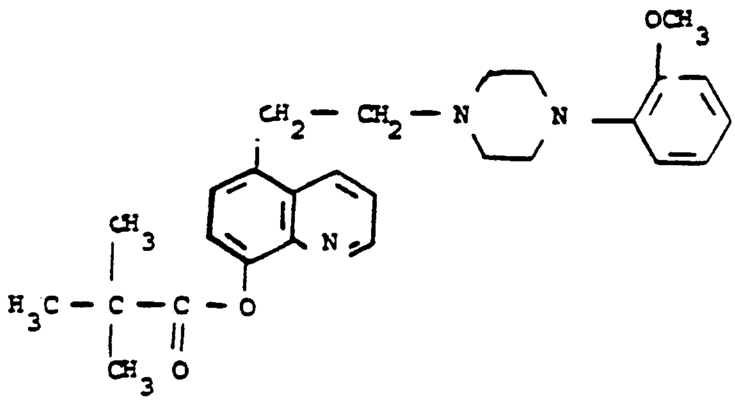

On laisse agiter pendant 48 heures à température ambiante un mélange de 7,27 g de {[(méthoxy-2 phényl)-4 pipérazinyl[-2 éthyl}-5 hydroxy-8 quinoléine dans 150 ml de tétrahydrofuranne anhydre en présence de 3,1 ml de triéthylamine et 5 ml de chlorure de pivaloyle. Après évaporation du tétrahydrofuranne, on reprend le résidu par le chloroforme et lave avec une solution à 10 % de Na $HCO_3$. Après évaporation du chloroforme, on obtient un résidu cristallin que l'on recristallise dans 80 ml d'isopropanol. On obtient finalement 6,45 g de cristaux de {[(méthoxy-2 phényl)-4 pipérazinyl]-2 éthyl}-5 pivaloyloxy-8 quinoléine, fondant à 115°C.

De la même façon a été préparé le dérivé objet de l'exemple suivant:

**Exemple 10:**

{[(méthoxy-2 phényl)-4 pipérazinyl]-2 éthyl}-5 méthylsulfonyloxy-8 quinoléine, isolée sous forme de résine.

**Exemple 11:**

diéthylphosphate d'hydroxy-8{[(méthoxy-2 phényl)-4 pipérazinyl]-2 éthyl}-5 quinoléine.

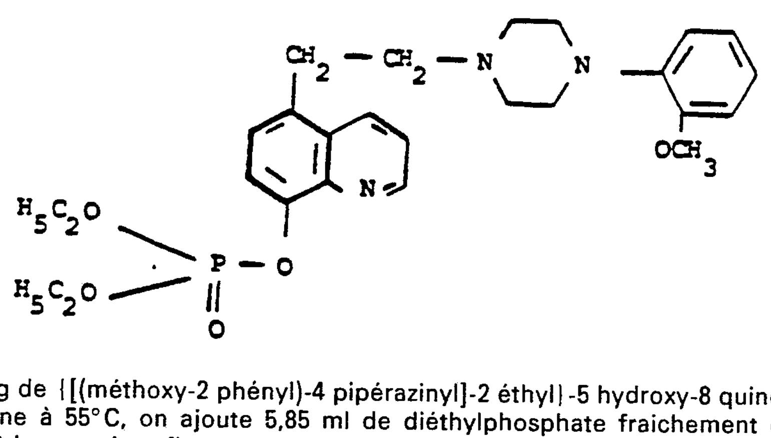

A une solution de 14,5 g de {[(méthoxy-2 phényl)-4 pipérazinyl]-2 éthyl}-5 hydroxy-8 quinoléine dans 320 ml de tétrachlorure de carbone à 55°C, on ajoute 5,85 ml de diéthylphosphate fraichement distillé et 6,28 ml de triéthylamine. Après 22 heures de reflux, on filtre l'insoluble et évapore le solvant sous pression réduite. On reprend le résidu dans 200 ml de cyclohexane à reflux, filtre l'insoluble gommeux et observe une cristallisation par refroidissement. On obtient 18 g de cristaux de diéthylphosphate d'hydroxy-8{[(méthoxy-2 phényl)-4 pipérazinyl]-2 éthyl}-5 quinoléine, fondant (capillaire) à 88 - 89°C (cyclohexane).

Selon la même méthode à été préparé le dérivé objet de l'exemple suivant:

**Exemple 12:**

Diéthylphosphate d'hydroxy-8{[(pyridyl-2)-4 pipérazinyl]-2 éthyl}-5 quinoléine, P.F.: 70°C (chlorure de méthylène).

**Exemple 13:**

{[(méthoxy-2 phényl)-4 pipérazinyl]-2 éthyl}-5 hydroxy-8 tétrahydro-1,2,3,4 quinoléine:

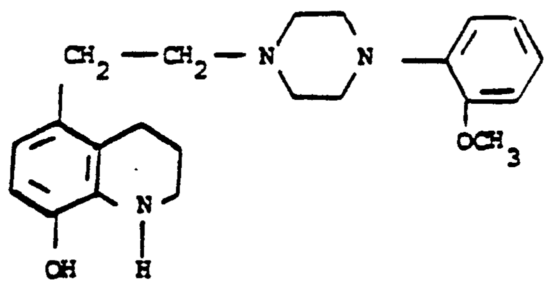

Dans un autoclave, on hydrogène pendant 20 heures à 85°C sous 125 atmosphères une solution de 3,65 g de {[(méthoxy-2 phényl)-4 pipérazinyl]-2 éthyl}-5 hydroxy-8 quinoléine, dans 50 ml d'éthanol, en présence de nickel de Raney. Ensuite, on filtre, et concentre à sec sous pression réduite. Le résidu est dissout dans l'isopropanol et on ajoute HCl gazeux de façon à former le dichlorhydrate. Les cristaux obtenus (3,8 g) sont alors traités avec une solution de $Na_2CO_3$ à 10 % et la base est extraite au chloroforme. Après évaporation du chloroforme on obtient des cristaux de {[(méthoxy-2 phényl)-4 pipérazinyl]-2 éthyl}-5 hydroxy-8 tétrahydro-1,2,3,4 quinoléine, fondant (capillaire) à 90°C.

**Exemples 14 à 17:**

Les dérivés suivants ont été préparés selon la méthode décrite dans l'exemple 13:

14) {[(méthoxy-2 phényl)-4 pipérazinyl]-2 éthyl}-5 méthylsulfonyloxy-8 tétrahydro-1,2,3,4 quinoléine, P.F. (capillaire) du fumarate correspondant: 184 - 186°C (éthanol).

15) {[(pyridyl-2)-4 pipérazinyl]-2 éthyl}-5 hydroxy-8 tétrahydro-1,2,3,4 quinoléine, produit amorphe.

16) {[(méthoxy-2 phényl)-4 pipérazinyl]-2 éthyl}-5 méthoxy-8 tétrahydro-1,2,3,4 quinoléine, P.F.: 88°C (chlorure de méthylène).

17) {[(pyridyl-2)-4 pipérazinyl]-2 éthyl}-5 méthoxy-8 tétrahydro-1,2,3,4 quinoléine, P.F.: 109°C (acétate d'éthyle).

**Exemple 18:**

acétyl-1{[(méthoxy-2 phényl)-4 pipérazinyl]-2 éthyl}-5 hydroxy-8 tétrahydro-1,2,3,4 quinoléine.

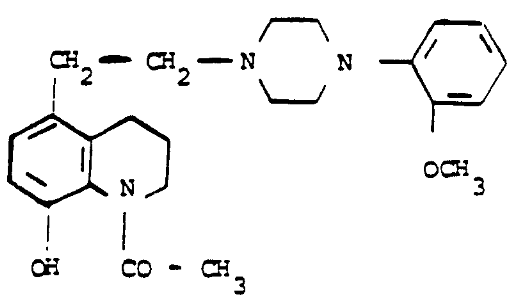

A une solution de 7,34 g de {[(méthoxy-2 phényl)-4 pipérazinyl]-2 éthyl}-5 hydroxy-8 tétrahydro-1,2,3,4 quinoléine dans 140 ml de tétrahydrofuranne anhydre contenant 2,8 ml de triéthylamine, on ajoute, à 5°C, une solution de 1,45 ml de chlorure d'acétyle dans 20 ml de tétrahydrofuranne. On agite pendant 2 heures à 5°C puis 1 heure à température ambiante. Ensuite, on filtre l'insoluble et concentre à sec sous pression réduite. Le résidu est purifié par chromato flash sur 750 g de silice (70 - 230 mesh) en éluant avec le mélange $CH_2Cl_2$-

$CH_3OH$ (95/5).

On obtient finalement 5 g de cristaux de chlorhydrate d'acétyl-1 {[(méthoxy-2 phényl)-4 pipérazinyl]-2 éthyl}-5 hydroxy-8 tétrahydro-1,2,3,4 quinoléine, fondant (capillaire) à 263 - 265° C.

**Exemples 19 à 29:**

Les dérivés suivants ont été préparés selon la méthode décrite dans l'exemple 18:

19) acétyl-1 {[(pyridyl-2)-4 pipérazinyl]-2 éthyl}-5 hydroxy-8 tétrahydro-1,2,3,4 quinoléine, P.F.: 168° C (acétate d'éthyle).

20) acétyl-1 {[(pyridyl-2)-4 pipérazinyl]-2 éthyl}-5 méthoxy-8 tétrahydro-1,2,3,4 quinoléine, P.F.: 111° C (acétate d'éthyle).

21) acétyl-1 {[(méthoxy-2 phényl)-4 pipérazinyl]-2 éthyl}-5 méthoxy-8 tétrahydro-1,2,3,4 quinoléine, P.F.: 112° C (acétate d'éthyle).

22) acétyl-1 {[(pyridyl-2)-4 pipérazinyl]-2 éthyl}-5 acétoxy-8 tétrahydro-1,2,3,4 quinoléine, P.F.: 129° C (chlorure de méthylène).

23) {[(méthoxy-2 phényl)-4 pipérazinyl]-2 éthyl}-5 dichloro-acétyloxy-8 tétrahydro-1,2,3,4 quinoléine.

24) {[(pyridyl-2)-4 pipérazinyl]-2 éthyl}-5 dichloro-acétyloxy-8 tétrahydro-1,2,3,4 quinoléine.

25) acétyl-1 {[(méthoxy-2 phényl)-4 pipérazinyl]-2 éthyl}-5 méthylsulfonyloxy-8 tétrahydro-1,2,3,4 quinoléine, P.F. (capillaire) du dichlorydrate correspondant: 189 - 197° C (isopropanol).

26) {[méthoxy-2 phényl)-4 pipérazinyl]-2 éthyl}-5 méthoxy-8 tétrahydro-1,2,3,4 quinoléine, produit amorphe.

27) acétyl-1 {[(pyridyl-2)-4 pipérazinyl]-2 éthyl}-5 méthylsulfonyloxy-8 tétrahydro-1,2,3,4 quinoléine, P.F. (capillaire) du dichlorhydrate correspondant: 199 - 201° C. (isopropanol/éther).

28) propionyl-1 {[(pyridyl-2)-4 pipérazinyl]-2 éthyl}-5 méthanesulfonyloxy-8 tétrahydro-1,2,3,4 quinoléine, P.F. (capillaire) du dichlorhydrate correspondant: 147 - 154° C (isopropanol/éther).

29) acétyl-1 {[(méthoxy-2 phényl)-4 pipérazinyl]-2 éthyl}-5 acétoxy-8 tétrahydro-1,2,3,4 quinoléine, P.F. (capillaire du dichlorhydrate correspondant: 249 - 252° C (avec décomposition) (isopropanol/éther).

**Exemple 30:**

méthyl-1 {[(méthoxy-2 phényl)-4 pipérazinyl]-2 éthyl}-5 hydroxy-8 tétrahydro-1,2,3,4 quinoléine.

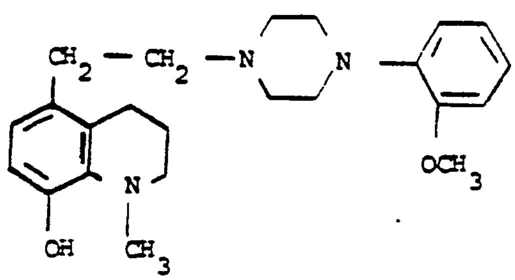

A une solution de 7,35 g de {[(méthoxy-2 phenyl)-4 pipérazinyl]-2 éthyl}-5 hydroxy-8 tétrahydro-1,2,3,4 quinoléine, dans 80 ml d'éthanol absolu, on ajoute à 5° C, 3,1 g d'iodure de méthyle dans 40 ml d'éthanol absolu. Au bout de 24 heures à température ambiante, on ajoute à nouveau 0,5 g d'iodure de méthyle et on maintient l'agitation pendant 24 heures. Ensuite, on concentre à sec sous pression réduite et reprend le résidu par une solution de $NaHCO_3$ à 10 % et du chloroforme. On décante et évapore à sec le chloroforme. Le résidu est purifié par chromato flash sur 700 g de silice en éluant avec un mélange $CH_2Cl_2$-$CH_3OH$ (95/5).

On obtient 2,5 g de produit résineux pur dont on forme le dichlorhydrate au sein de l'isopropanol. On recueille finalement 2,3 g de cristaux blancs fondant (capillaire) à 168 - 175° C.

De la même façon à été préparé le dérivé de l'exemple suivant:

**Exemple 31:**

méthyl-1 {[(pyridyl-2)-4 pipérazinyl]-2 éthyl}-5 hydroxy-8 tétrahydro-1,2,3,4 quinoléine.

**Exemple 32:**

méthyl-1 {[(méthoxy-2 phényl)-4 pipérazinyl]-2 éthyl}-5 méthoxy-8 tétrahydro-1,2,3,4 quinoléine.

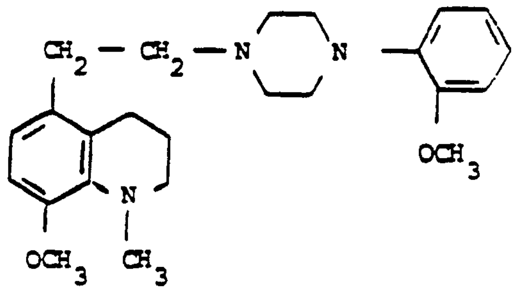

a) Première méthode:

En opérant comme dans l'exemple 5, à partir de 3 g de méthyl-1 {[(méthoxy-2 phényl)-4 pipérazinyl]-2 éthyl}-5 hydroxy-8 tétrahydro-1,2,3,4 quinoléine, on obtient 1,9 g de cristaux de méthyl-1 {[(méthoxy-2 phényl)-4 pipérazinyl]-2 éthyl}-5 méthoxy-8 quinoléine, fondant (capillaire) à 94° C.

En opérant de la même manière, on à également préparée la méthyl-1 {[(pyridyl-2)-4 pipérazinyl]-2 éthyl}-5 méthoxy-8 tétrahydro-1,2,3,4 quinoléine déjà décrite dans l'exemple 7.

b) Deuxième méthode:

La méthyl-1 {[(méthoxy-2 phényl)-4 pipérazinyl]-2 ethyl}-5 méthoxy-8 tétrahydro-1,2,3,4 quinoléine à également été préparée par réduction au moyen de Li Al $H_4$ dans le tétrahydrofuranne à ébullition de la formyl-1 {[(méthoxy-2 phényl)-4 pipérazinyl]-2 éthyl}-5 méthoxy-8 tétrahydro-1,2,3,4 quinoléine, objet de l'exemple suivant:

**Exemple 33:**

Formyl-1 {[(méthoxy-2 phényl)-4 pipérazinyl]-2 éthyl}-5 méthoxy-8 tétrahydro-1,2,3,4 quinoléine, P.F.: 110° C (acétate d'éthyle). Ce dérivé à été préparé par formylation avec HCO OH à 98 % à l'ébullition de la {[(méthoxy-2 phényl)-4 pipérazinyl]-2 éthyl}-5 méthoxy-8 tétrahydro-1,2,3,4 quinoléine, P.F.: 88° C.

**Exemples 34 - 35:**

En opérant comme dans l'exemple 32 b), à partir des acétyl-1 {[(pyridyl-2)-4 pipérazinyl]-2 éthyl}-5 méthoxy-8 tétrahydro-1,2,3,4 quinoléine, et acétyl-1 {[(méthoxy-2 phényl)-4 pipérazinyl]-2 éthyl}-5 méthoxy-8 tétrahydro-1,2,3,4 quinoléine, on obtient respectivement les dérivés suivants:

34) éthyl-1 {[(pyridyl-2)-4 pipérazinyl]-2 éthyl}-5 méthoxy-8 tétrahydro-1,2,3,4 quinoléine.

35) éthyl-1 {[(méthoxy-2 phényl)-4 pipérazinyl]-2 éthyl}-5 méthoxy-8 tétrahydro-1,2,3,4 quinoléine.

**Revendications** pour les Etats Contractants: BE, CH, DE, FR, GB, IT, LI, LU, NL, SE

1. Les dérivés de la quinoléine de formule générale I:

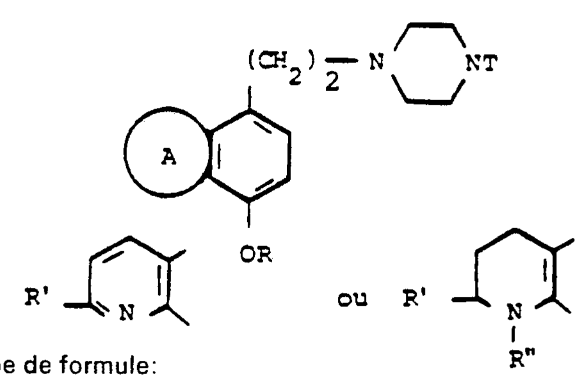

I

ou

dans laquelle:

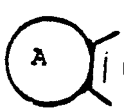 représente un groupe de formule:

dans lesquelles:
R' représente un atome d'hydrogène ou un radical méthyle;
R et R", identiques ou différents représentent chacun:
- un atome d'hydrogéne,
- un radical alcoyle ayant de 1 à 5 atomes de carbone,
- un radical acyle ayant de 1 à 5 atomes de carbone, comportant éventuellement des atomes d'halogène ou des groupes amino,
- un radical alkylsulfonyle renfermant de 1 à 3 atomes de carbone, ou
- un radical diéthylphosphonyle;
T est un radical méthoxy-2 phényle ou pyridyle-2.

2. Les sels des composés de la revendication 1 avec des acides appropriés.

3. Les sels selon la revendication 2 qui sont physiologiquement tolérables.

4. La {[(méthoxy-2 phényl)-4 pipérazinyl]-2 éthyl}-5 méthoxy-8 quinoléine, et son fumarate.

5. La {[(méthoxy-2 phényl)-4 pipérazinyl]-2 éthyl}-5 méthylsulfonyloxy-8 tétrahydro-1,2,3,4 quinoléine et son fumarate.

6. L'acétyl-1{[(méthoxy-2 phényl)-4 pipérazinyl]-2 éthyl}-5 hydroxy-8 tétrahydro-1,2,3,4 quinoléine, et son chlorhydrate.

7. L'acétyl-1{[(pyridyl-2)-4 pipérazinyl]-2 éthyl}-5 hydroxy-8 tétrahydro-1,2,3,4 quinoléine.

8. L'acétyl-1{[(pyridyl-2)-4 piperazinyl]-2 éthyl}-5 méthoxy-8 tétrahydro-1,2,3,4 quinoléine.

9. l'acétyl-1{[(méthoxy-2 phényl)-4 pipérazinyl]-2 éthyl}-5 méthoxy-8 tétrahydro-1,2,3,4 quinoléine.

10. La méthyl-1{[(méthoxy-2 phényl)-4 piperazinyl]-2 éthyl}-5 hydroxy-8 tétrahydro-1,2,3,4 quinoléine, et son dichlorhydrate.

11. l'acétyl-1{[(méthoxy-2 phényl)-4 pipérazinyl]-2 éthyl}-5 méthylsulfonyloxy-8 tétrahydro-1,2,3,4 quinoléine, et son dichlorhydrate.

12. la formyl-1{[(méthoxy-2 phényl)-4 pipérazinyl]-2 éthyl}-5 méthoxy-8 tétrahydro-1,2,3,4 quinoléine.

13. Le procédé de préparation des composés de la revendication 1 répondant à la formule générale I':

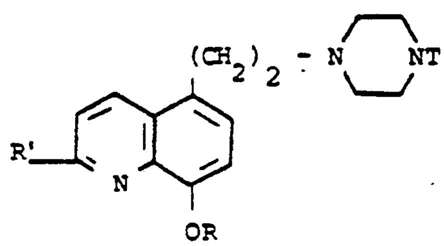

I'

dans laquelle:
R, R' et T sont tels que définis dans la revendication 1, caractérisé en ce que:
- l'on condense un dérivé halogéné de formule générale II:

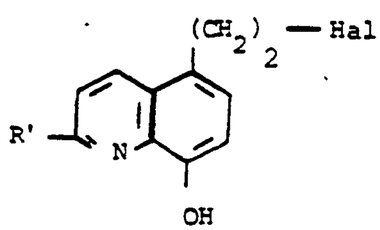

II

dans laquelle R' à la signification définie dans la revendication 1 et Hal représente un atome de chlore ou de brome, avec une pipérazine N-mono substituée de formule générale III

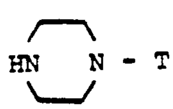

III

dans laquelle T à la signification définie dans la revendication 1, puis l'on traite le dérivé ainsi obtenu de formule générale IV:

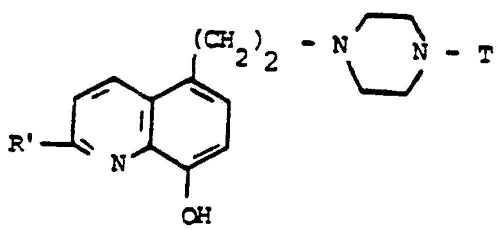

IV

dans laquelle R' et T ont les significations définies, dans la revendication 1, avec un dérivé de formule générale V:

HZ (V)

dans laquelle R prend la signification définie, dans la revendication 1, et Z représente, selon les valeurs de R, un atome d'halogène ou un des radicaux, $-PO_3H$, -COCl, $SO_2$ Cl ou Ar-$SO_3$ dans lequel Ar représente un groupe phényle éventuellement substitué par un ou plusieurs radicaux alcoyle ayant de 1 à 5 atomes de carbone.

14. Le procédé de préparation, selon la revendication 13, caractérisé en ce que la condensation des dérivés II et III est effectuée dans un solvant polaire, à une température comprise entre 60 et 120°C, en présence d'un accepteur de l'hydracide formé au cours de la réaction.

15. Le procédé de préparation des composés de la revendication 1, répondant à la formule générale I":

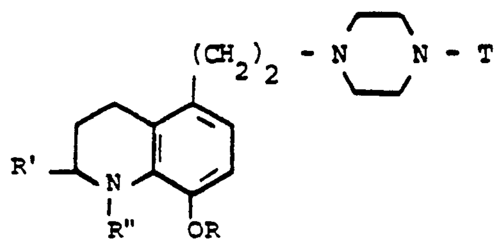

I"

dans laquelle R, R', R" et T ont les significations définies dans la revendication 1, caractérisé en ce que:

- l'on réduit le dérivé de formule générale I':

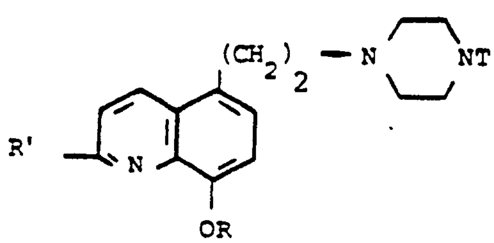

I'

dans laquelle R, R' et T sont tels que définis dans la revendication 1, en un dérivé de formule générale II':

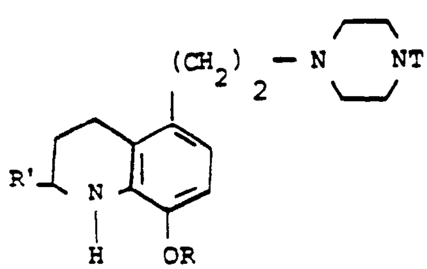

II'

que l'on traite ensuite par un réactif de formule générale III'

R" - Z III'

dans laquelle R" a la signification définie dans la revendication 1 et Z est tel que défini dans la revendication 13.

16. Le procédé de préparation selon la revendication 15, caractérisé en ce que la réduction du dérivé (I') en dérivé (II') se fait par l'hydrogène en présence d'un catalyseur du 8ème groupe, dans un solvant polaire sous une pression de $3 \cdot 10^6$ à $11 \cdot 10^6$ Pa à une température comprise entre 50 et 80°C.

17. Les compositions pharmaceutiques contenant comme principe actif un composé selon les revendications 1 et 3 à 12, avec les excipients pharmaceutiques appropriés.

18. Les compositions pharmaceutiques selon la revendication 17 présentées sous une forme convenant pour le traitement des troubles liés à l'hypoxémie et à l'insuffisance métabolique énergétique notamment lors du vieillissement cérébral.

**Revendication** pour l'Etat contractant: AT

Procédé de préparation des dérivés de la quinoléine de formule générale I:

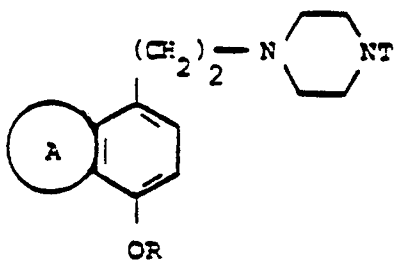

(I)

dans laquelle:

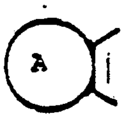 représente un groupe de formule:

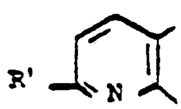

ou

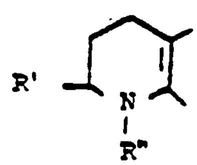

dans lesquelles:

R' représente un atome d'hydrogène ou un radical méthyle;

R et R", identiques ou différents représentent chacun:

- un atome d'hydrogène,
- un radical alcoyle ayant de 1 à 5 atomes de carbone,
- un radical acyle ayant de 1 à 5 atomes de carbone, comportant éventuellement des atomes d'halogène ou des groupes amino,
- un radical alkylsulfonyle renfermant de 1 à 3 atomes de carbone, ou
- un radical diéthylphosphonyle; et

T est un radical méthoxy-2 phényle ou pyridyle-2.

- et de leurs sels d'addition avec des acides appropries;

caractérisé en ce que:

a) l'on condense un dérivé halogéné de formule générale II:

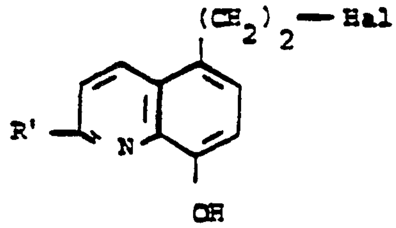

(II)

dans laquelle R' à la signification définie ci-dessus et Hal représente un atome de chlore ou de brome, avec une pipérazine N-mono substituée de formule générale III

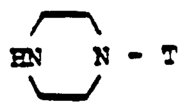

(III)

dans laquelle T a la signification précédemment définie, puis l'on traite le dérivé ainsi obtenu de formule générale IV:

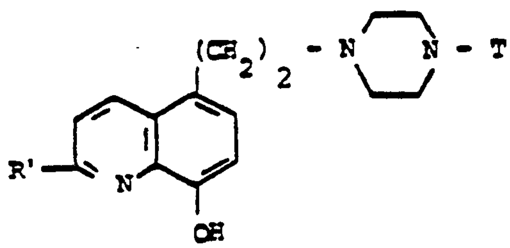

(IV)

dans laquelle R' et T ont les significations définies précédemment, avec un dérivé de formule générale V:

RZ (V)

dans laquelle R prend la signification définie précédemment et Z représente, selon les valeurs de R, un atome d'halogène ou un des radicaux, $-PO_3H$, -COCl, $SO_2$ Cl ou Ar-$SO_3$ dans lequel Ar représente un groupe phényle éventuellement substitué par un ou plusieurs radicaux alcoyle ayant de 1 à 5 atomes de carbone, pour obtenir les composes de formule générale I':

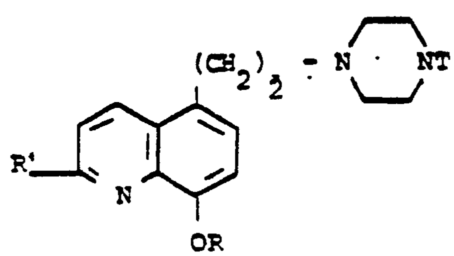

(I')

dans laquelle:

R, R' et T sont tels que précédemment définis, et

b) l'on réduit le dérivé de formule générale I':

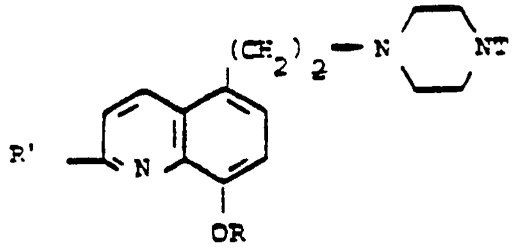

(I')

dans laquelle R, R' et T sont tels que définis précédemment en un dérivé de formule générale II':

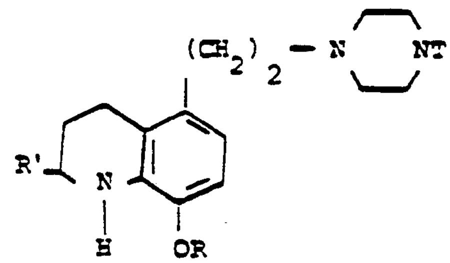

(II')

que l'on traite ensuite par un réactif de formule générale III'

R" - Z (III')

dans laquelle R" et Z ont les significations définies précédemment pour obtenir les composés de formule générale I":

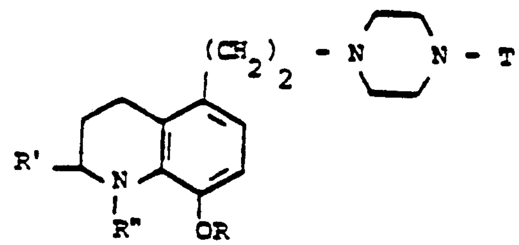

(I")

dans laquelle R, R', R" et T ont les significations précédemment définies,

et si on le désire, on traite les dérivés (I') et (I") ainsi obtenus, avec des acides appropriés pour donner les sels d'addition correspondants.

**Patentansprüche** für die Vertragsstaaten: BE, CH, DE, FR, GB, IT, LI, LU, NL SE

1. Chinolinderivate der allgemmeinen Formel I

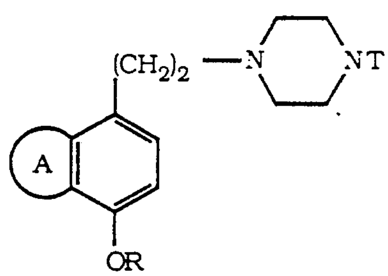

I

in der

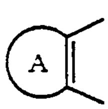 eine Gruppe der Formel 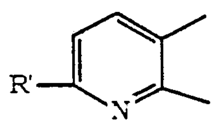 oder 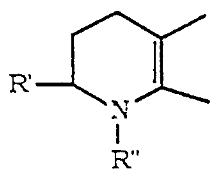

darstellt, vorin

R' ein Wasserstoffatom oder eine Methylgruppe;

R und R", die gleichartig oder verschieden sein können

- Wasserstoffatome,
- Alkylgruppen mit 1 bis 5 Kohlenstoffatomen,
- Acylgruppen mit 1 bis 5 Kohlenstoffatomen, die gegebenenfalls Halogenatome oder Aminogruppen tragen können
- Alkylsulfonylgruppen mit 1 bis 3 Kohlenstoffatomen oder
- Diethylphosphonylgruppen; und

T eine 2-Methoxy-phenyl- oder 2-Pyridylgruppe bedeuten.

2. Die Salze der Verbindung nach Anspruch 1 mit geeigneten Säuren.

3. Die Salze nach Anspruch 2, die physiologisch verträglich sind.

4. 5-{2-[4-(2-methoxy-phenyl)-piperazinyl]-ethyl}-8-methoxy-chinolin und dessen Fumarat.

5. 5-{2-[4-(2-methoxy-phenyl)-piperazinyl]-ethyl)-8-methylsulfonyloxy-1,2,3,4-tetrahydrochinolin und dessen Fumarat.

6. 1-Acetyl-5-{2-[4-(2-methoxy-phenyl)-piperazinyl]-ethyl}-8-hydroxy-1,2,3,4-tetrahydrochinolin und dessen Hydrochlorid.

7. 1-Acetyl-5-{2-[4-(2-pyridyl)-piperazinyl]-ethyl}-8-hydroxy-1,2,3,4-tetrahydro-chinolin.

8. 1-Acetyl-5-{2-[4-(2-pyridyl)-piperazinyl]-ethyl}-8-methoxy-1,2,3,4-tetrahydrochinolin.

9. 1-Acetyl-5-{2-[4-(2-methoxy-phenyl)-piperazinyl]-ethyl}-8-methoxy-1,2,3,4-tetrahydrochinolin.

10. 1-Methyl-5-{2-4-(2-methoxy-phenyl)-piperazinyl]-ethyl}-8-hydroxy-1,2,3,4-tetrahydrochinolin und dessen Dihydrochlorid.

11. 1-Acetyl-5-{2-[4-(2-methoxy-phenyl)-piperazinyl]-ethyl}-8-methylsulfonyloxy-1,2.3,4-tetrahydrochinolin und dessen Dihydrochlorid.

12. 1-Formyl-5-{2-[4-(2-methoxy-phenyl)-piperazinyl]-ethyl}-8-methoxy-1,2,3,4-tetrahydrochinolin.

13. Verfahren zur Herstellung der Verbindungen nach Anspruch 1 der allgemeinen Formel I'

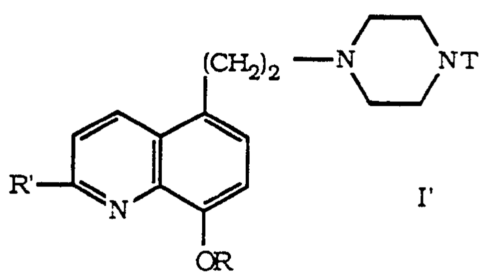

I'

worin

R, R' und T die in Anspruch 1 angegebenen Bedeutungen besitzen, dadurch gekennzeichnet, daß man

- ein Halogenderivat der allgemeinen Formel II

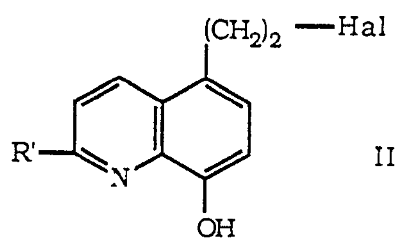

II

in der R' die in Anspruch 1 angegebenen Bedeutungen besitzt und Hal ein Chlor- oder Bromatom darstellt mit einem N-monosubstituierten Piperazin der allgemeinen Formel III

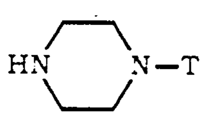

III

worin T die in Anspruch 1 angegebenen Bedeutungen besitzt, kondensiert und das in diese Weise erhaltene Derivat der allgemeinen Formel IV

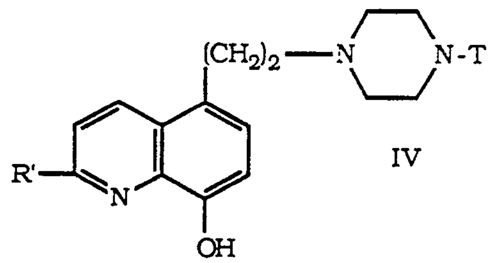

IV

in der R'und T die in Anspruch 1 angegebenen Bedeutungen besitzen, mit einem Derivat der allgemeinen Formel V behandelt

RZ V

in der R die in Anspruch 1 angegebenen Bedeutungen besitzt und Z in Abhängigkeit von der Bedeutung von R ein Halogenatom oder einen der Reste der Formeln $-PO_3H$, -COCl, $SO_2Cl$ oder $Ar-SO_3$, worin Ar eine gegebenenfalls durch eine oder mehrere Alkylgruppen mit 1 bis 5 Kohlenstoffatomen substituierte Phenylgruppe darstellt, bedeutet.

14. Verfahren nach Anspruch 13, dadurch gekennzeichnet, daß die Kondensation der Derivate der Formeln II und III in einem polaren Lösungsmittel bei einer Temperatur zwischen 60 und 120°C in Gegenwart eines Akzeptors für die im Verlaufe der Reaktion gebildete Halogenwasserstoffsäure durch geführt wird.

15. Verfahren zur Herstellung von Verbindungen nach Anspruch 1 der allgemeinen Formel I''

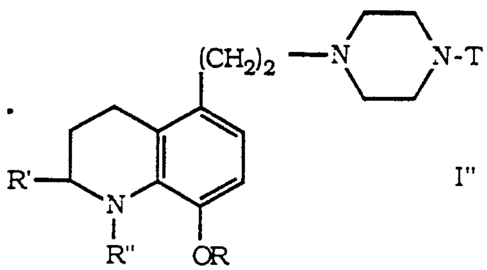

I''

in der R, R', R'' und T die in Ansrpuch 1 angegebenen Bedeutungen besitzen, dadurch gekennzeichnet, daß man das Derivat der allgemeinen Formel I'

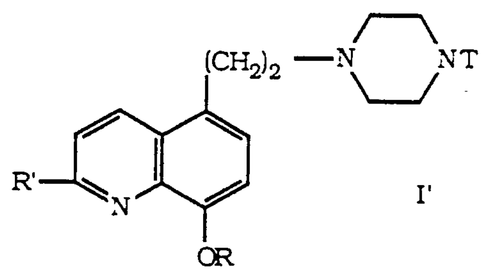

I'

in der R, R' und T die in Anspruch 1 angegebenen Bedeutungen besitzen, zu einem Derivat der allgemeinen Formel II'

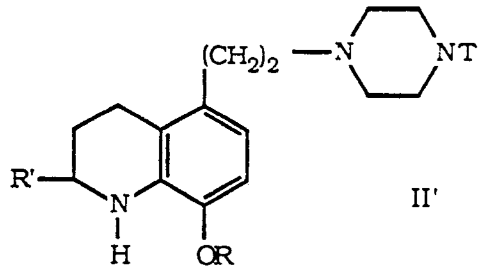

II'

reduziert, welches man anschließend mit einem Reagens der allgemeinen Formel III'

R" - Z        III'

in der R" die in Anspruch 1 angegebenen Bedeutungen besitzt und Z die in Anspruch 13 angegebenen Bedeutungen aufweist, behandelt.

16. Verfahren nach Anspruch 15, <u>dadurch gekennzeichnet</u>, daß die Reduktion des Derivats der Formel I' zu dem Derivat der Formel II' mit Wasserstoff im Gegenwart eines Katalysators aus der achten Gruppe der Periodensystems in einem polaren Lösungsmittel bei einem Druck von $3 \cdot 10^6$ bis $11 \cdot 10^6$Pa und bei einer Temperatur zwischen 50 und 80° C bewirkt wird.

17. Pharmazeutische Zubereitungen enthaltend als Wirkstoff eine Verbindung nach einem der Ansprüche 1 und 3 bis 12 zusammen mit geeigneten pharmazeutischen Hilfsstoffen.

18. Pharmazeutische Zubereitung nach Anspruch 17, <u>dadurch gekennzeichnet</u>, daß sie in einer Form vorliegen, die zur Behandlung von Störungen geeignet ist die durch Hypoxämie und Energiestoffwechselinsuffizienzien insbesondere bei der Alterung des Gehirns verursacht werden.

**Patentanspruch** für den Vertragsstaat: AT

Verfahren zur Herstellung von Chinolinderivaten der allgemeinen Formel I

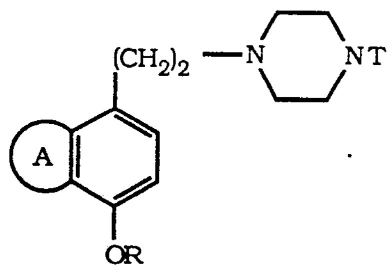

I

in der

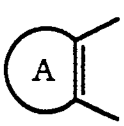 ein Gruppe der Formel 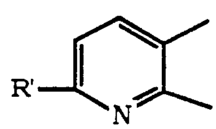 oder 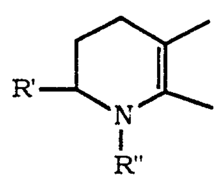

darstell worin

R' ein Wasserstoffatom oder eine Methylgruppe;

R und R", die gleichartig oder verschieden sein können

- Wasserstoffatome,
- Alkylgruppen mit 1 bis 5 Kohlenstoffatomen,
- Alcylgruppen mit 1 bis 5 Kohlenstoffatomen, die gegebenenfalls Halogenatome oder Aminogruppen tragen

können

- Alkylsulfonylgruppen mit 1 bis 3 Kohlenstoffatomen oder
- Diethylphosphonylgruppen; und

T eine 2-Methoxy-phenyl- oder 2-Pyridylgruppe bedeuten, und deren Additionsalzen mit geeigneten Säuren, dadurch gekennzeichnet, daß man

a) ein Halogenderivat der allgemeinen Formel II

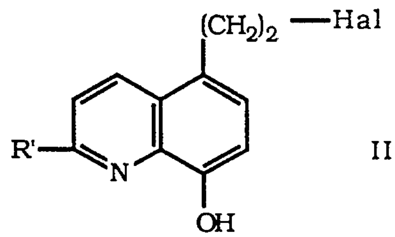

II

in der R' die oben angegebenen Bedeutungen besitzt und Hal ein Chlor- oder Bromatom darstellt, mit einem N-monosubstituierten Piperazin der allgemeinen Formel III

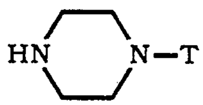

III

worin T die oben angegebenen Bedeutungen besitzt, kondensiert und dann das in diese Weise erhaltene Derivat der allgemeinen Formel IV

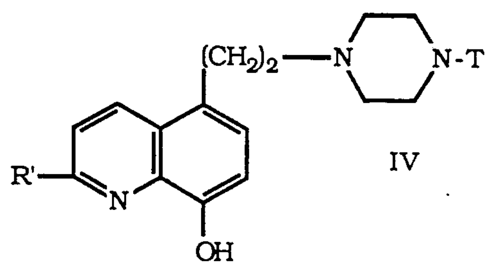

IV

worin R' und T die oben angegebenen Bedeutungen besitzen, mit einem Derivat der allgemeinen Formel V behandelt

RZ . V

worin R die oben angegebenen Bedeutungen besitzt und Z in Abhängigkeit von der Bedeutung von R ein Halogenatom oder ein Rest der Formeln $-PO_3H$, - COCl, $SO_2Cl$ oder $Ar-SO_3$, worin Ar eine gegebenenfalls durch eine oder mehrere Alkylgruppen mit 1 bis 5 Kohlenstoffatomen substituierte Phenylgruppe darstellt, bedeutet, zur Bildung von Verbindungen der allgemeinen Formel I'

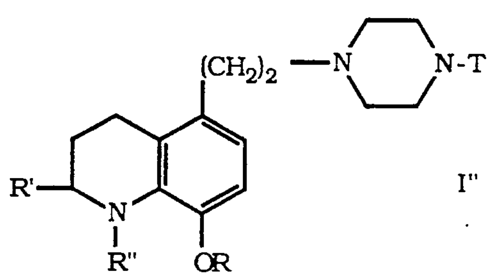

I"

in der

R, R' und T die oben angegebenen Bedeutungen besitzen, und

b) das Derivat der allgemeinen Formel I'

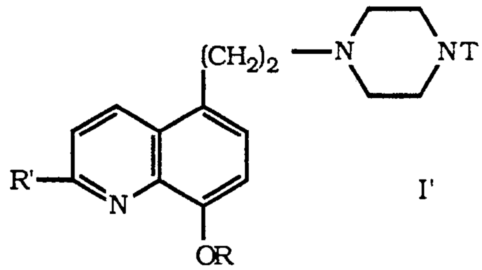

I'

worin R, R' und T die oben angegebenen Bedeutungen besitzen, zu einem Derivat der allgemeinen Formel II'

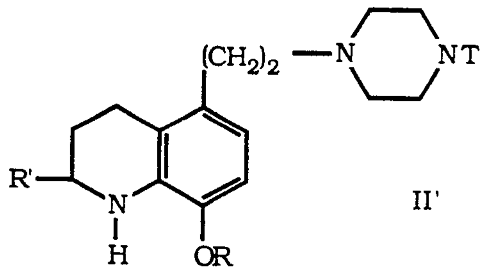

II'

reduziert, welches anschließend mit einem Reagens der allgemeinen Formel III'

R" - Z III'

in Der R" und Z die oben angegebenen Bedeutungen besitzen, behandelt zur Bildung von Verbindungen der allgemeinen Formel I"

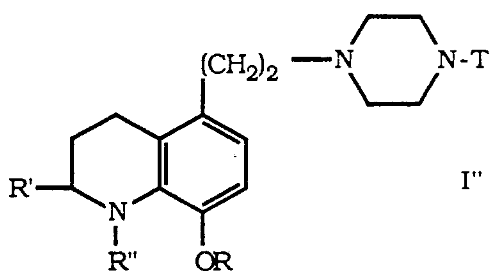

I"

in der R, R', R" und T die oben angegebenen Bedeutungen besitzen, und gewünschtenfalls die in dieser Weise erhaltenen Derivate der Formeln I' und I" mit geeigneten Säuren zur Bildung der entsprechenden Additionssalze behandelt.

**Claims** for the Contracting States: BE, CH, DE, FR, GB, IT, LI, LU, NL, SE

1. Quinoline derivatives of the general formula I:

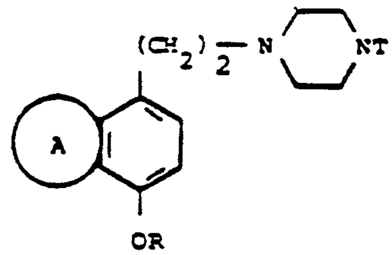

I

in which

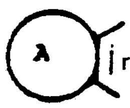 represents a group of the formula:

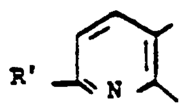

or

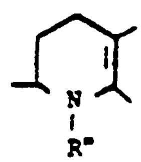

in which
R′ represents a hydrogen atom or a methyl radical,
R and R″, which may be the same or different, each represents a hydrogen atom, an alkyl radical having from 1 to 5 carbon atoms, an acyl radical having from 1 to 5 carbon atoms which may contain halogen atoms or amino groups, an alkylsulphonyl radical containing from 1 to 3 carbon atoms, or a diethylphosphonyl radical,
T is a 2-methoxyphenyl or 2-pyridyl radical.

2. Salts of compounds of claim 1 with appropriate acids.
3. Salts according to claim 2 that are physiologically tolerable.
4. 5-{2-[4-(2-methoxyphenyl)-piperazinyl]-ethyl}-8-methoxyquinoline and its fumarate.
5. 5-{2-[4-(2-methoxyphenyl)-piperazinyl]-ethyl}-8-methylsulphonyloxy-1,2,3,4-tetrahydroquinoline and its fumarate.
6. 1-acetyl-5-{2-[4-(2-methoxyphenyl)-piperazinyl]-ethyl}-8-hydroxy-1,2,3,4-tetrahydroquinoline and its hydrochloride.
7. 1-acetyl-5-{2-[4-(2-pyridyl)-piperazinyl]-ethyl}-8-hydroxy-1,2,3,4-tetrahydroquinoline.
8. 1-acetyl-5-{2-[4-(2-pyridyl)-piperazinyl]-ethyl}-8-methoxy-1,2,3,4-tetrahydroquinoline.
9. 1-acetyl-5-{2-[4-(2-methoxyphenyl)-piperazinyl]-ethyl}-8-methoxy-1,2,3,4-tetrahydroquinoline.
10. 1-methyl-5-{2-[4-(2-methoxyphenyl)-piperazinyl]-ethyl}-8-hydroxy-1,2,3,4-tetrahydroquinoline and its dihydrochloride.
11. 1-acetyl-5-{2-[4-(2-methoxyphenyl)-piperazinyl]-ethyl}-8-methylsulphonyloxy-1,2,3,4-tetrahydroquinoline and its dihydrochloride.
12. 1-formyl-5-{2-[4-(2-methoxyphenyl)-piperazinyl]-ethyl}-8-methoxy-1,2,3,4-tetrahydroquinoline.
13. Process for the preparation of compounds of claim 1 corresponding to the general formula I′

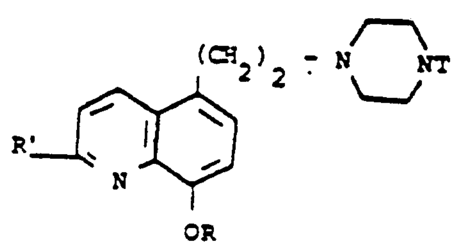

I′

in which R, R′ and T are as defined in claim 1,
- characterised in that
a halogen derivative of the general formula II:

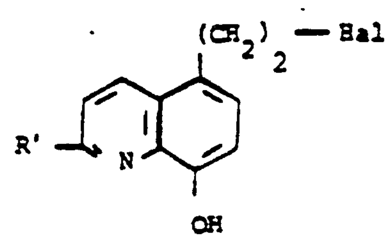

II

in which R′ has the meaning given in claim 1, and Hal represents a chlorine or bromine atom, is condensed with an N-mono-substituted piperazine of the general formula III

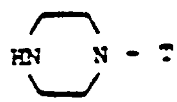

III

in which T has the meaning given in claim 1,

- the derivative so obtained of the general formula IV:

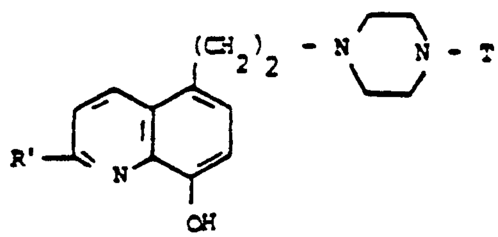

IV

in which R′ and T have the meanings given in claim 1, is then treated with a derivative of the general formula V:

RZ V

in which R has the meaning given in claim 1 and Z represents, depending on the meanings of R, a halogen atom or one of the radicals $-PO_3H$, $-COCl$, $SO_2Cl$, or $Ar-SO_3$ in which Ar represents a phenyl group which may be substituted by one or more alkyl radicals having from 1 to 5 carbon atoms.

14. Preparation process according to claim 13, characterised in that the condensation of derivatives II and III is carried out in a polar solvent at a temperature between 60 and 120°C in the presence of a hydracid acceptor formed during the reaction.

15. Process for the preparation of compounds of claim 1 corresponding to the general formula I″:

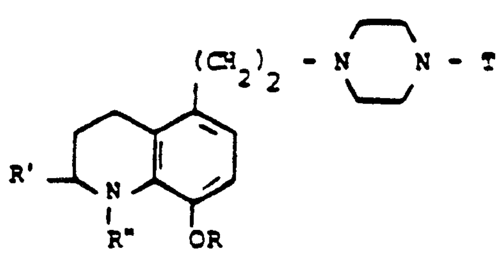

I″

in which R, R′, R″ and T have the meanings given in claim 1,

- characterised in that:

the derivative of the general formula I′:

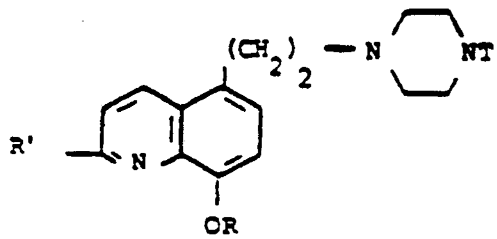

I'

in which R, R' and T are as defined in claim 1, is reduced to a derivative of the general formula II':

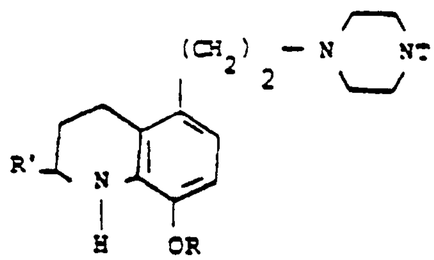

II'

which is then treated with a reactant of the general formula III'

$R'' - Z$ III'

in which R" has the meaning given in claim 1 and Z is as defined in claim 13.

16. Preparation process according to claim 15, characterised in that the reduction of the derivative (I') to derivative (II') is carried out by hydrogen in the presence of a catalyst from group 8 in a polar solvent under a pressure of from $3 \cdot 10^6$ Pa to $11 \cdot 10^6$ Pa, at a temperature between 50 and 80° C.

17. Pharmaceutical compositions containing as active ingredient a compound according to claims 1 and 3 to 12 with appropriate pharmaceutical excipients.

18. Pharmaceutical compositions according to claim 17, presented in a form suitable for the treatment of problems connected with hypoxemia and with insufficiency in energy metabolism, especially in the case of cerebral ageing.

**Claim** for the Contracting State: AT

Process for the preparation of quinoline derivatives of the general formula I:

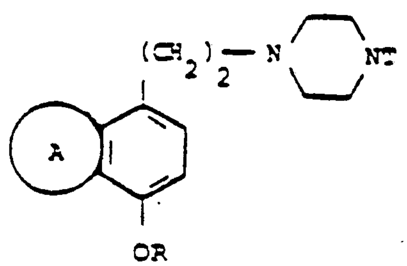

in which (I)

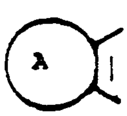 represents a group of the formula:

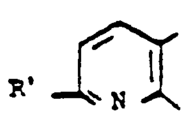 or 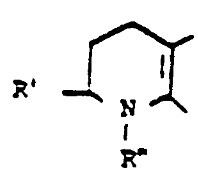

in which

R′ represents a hydrogen atom or a methyl radical,

R and R″, which may be the same or different, each represents a hydrogen atom, an alkyl radical having from 1 to 5 carbon atoms, an acyl radical having from 1 to 5 carbon atoms which may contain halogen atoms or amino groups, an alkylsulphonyl radical containing from 1 to 3 carbon atoms, or a diethylphosphonyl radical, and

T is a 2-methoxyphenyl or 2-pyridyl radical, and the addition salts thereof with appropriate acids; characterised in that

a) a halogen derivative of the general formula II:

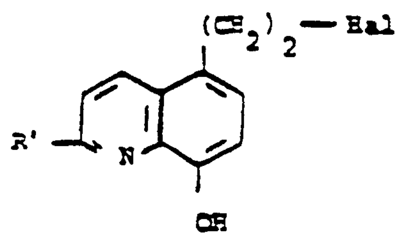

(II)

in which R′ has the meaning given above, and Hal represents a chlorine or bromine atom, is condensed with an N-mono-substituted piperazine of the general formula III

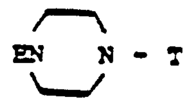

(III)

in which T has the meaning given hereinbefore, the derivative so obtained of the general formula IV:

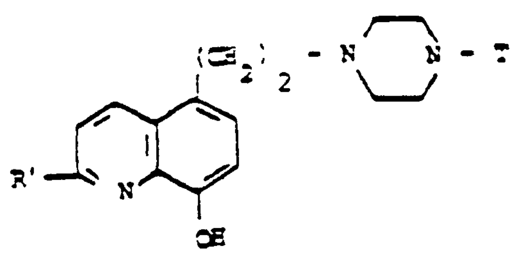

(IV)

in which R′ and T have the meanings given hereinbefore, is then treated with a derivative of the general formula V:

RZ (V)

in which R has the meaning given hereinbefore and Z represents, depending on the meanings of R, a halogen atom or one of the radicals $-PO_3H$, $-COCl$, $SO_2Cl$, or $Ar-SO_3$ in which Ar represents a phenyl group which may be substituted by one or more alkyl radicals having from 1 to 5 carbon atoms, to obtain compounds of the general formula I′

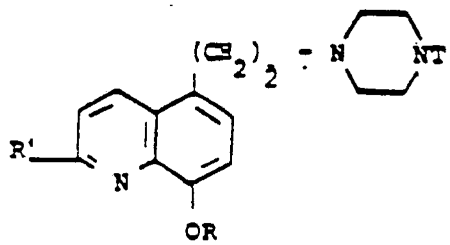

(I′)

in which R, R' and T are as defined hereinbefore, and
b) the derivative of the general formula I'

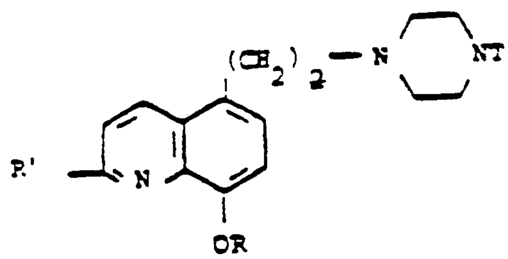

(I')

in which R, R' and T are as defined hereinbefore, is reduced to a derivative of the general formula II':

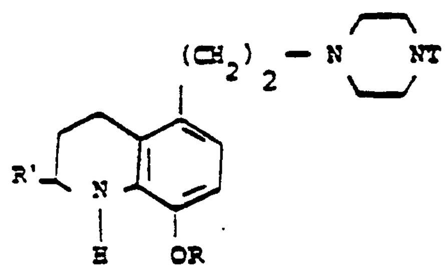

(II')

which is then treated with a reactant of the general formula III'

R" - Z (III')

in which R" and Z are as defined hereinbefore, to obtain compounds of the general formula I",

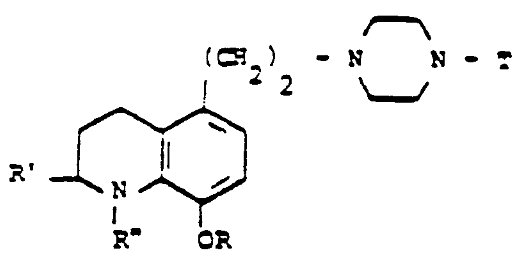

(I")

in which R, R', R" and T are as defined hereinbefore, and, if desired, the derivatives (I') and (I") so obtained are treated with appropriate acids to yield corresponding addition salts.